(19) Europäisches Patentamt
European Patent Office
Office européen des brevets

(11) Publication number: 0 297 858 A2

(12) **EUROPEAN PATENT APPLICATION**

(21) Application number: 88305925.5

(22) Date of filing: 28.06.88

(51) Int. Cl.⁴: C 07 D 471/08
A 61 K 31/47
//(C07D471/08,221:00,209:00),
(C07D471/08,245:00,221:00)

(30) Priority: 02.07.87 PC /US87/01583

(43) Date of publication of application:
04.01.89 Bulletin 89/01

(84) Designated Contracting States:
AT BE CH DE ES FR GB GR IT LI LU NL SE

(71) Applicant: PFIZER INC.
235 East 42nd Street
New York, N.Y. 10017 (US)

(72) Inventor: Fox, Edward Kleinman
544A Shennecossett Road
Groton Connecticut (US)

(74) Representative: Bradbrook, Geoffrey William et al
PFIZER LIMITED Ramsgate Road
Sandwich Kent (GB)

Claims for the following Contracting States: ES + GR.

(54) **Bridged-diazabicycloalkyl quinolone carboxylic acids and esters.**

(57) Compounds of the formula

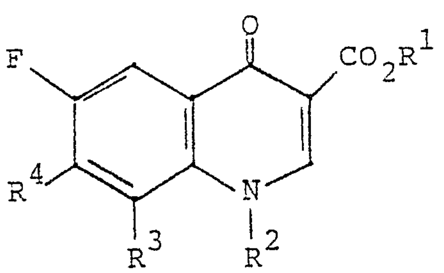

and pharmaceutically acceptable acid addition salts thereof, wherein

$R^1$ is hydrogen, $(C_1\text{-}C_6)$alkyl, or a pharmaceutically acceptable cation;

$R^2$ is ethyl, fluoroethyl, p-fluorophenyl, p-hydroxyphenyl or cyclopropyl;

$R^3$ is fluorine or hydrogen; and

$R^4$ is

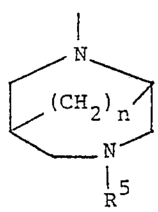

(a) or

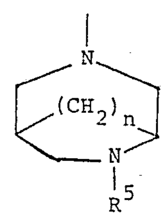

(b)

wherein n is 1 or 2 and $R^5$ is hydrogen or $(C_1\text{-}C_3)$alkyl are disclosed. Also disclosed are antibacterial pharmaceutical compositions comprising the foregoing compounds, methods of using the compounds in treating bacterial infections, and intermediates for the preparation of the compounds.



Bundesdruckerei Berlin

## Description

### BRIDGED-DIAZABICYCLOALKYL QUINOLONE CARBOXYLIC ACIDS AND ESTERS

This invention relates to substituted bridged-diazabicycloalkyl quinolone carboxylic acids and esters, antibacterial compositions containing said compounds, methods of using said compounds, and intermediates for the preparation of said compounds.

European Patent Application 86307045.4 (Publication No. 0215650), United States Patent 4,571,396 and European Patent Application 83305148.5 (Publication No. 0106489) refer to bridged-diazabicycloalkyl quinolone carboxylic acids and esters and antibacterial compositions containing those compounds.

The present invention relates to compounds of the formula

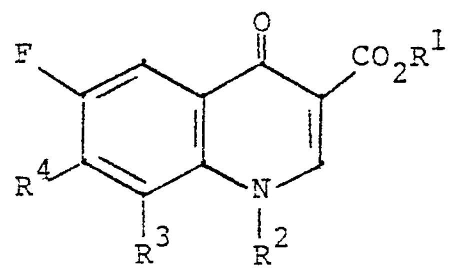

I

and pharmaceutically acceptable acid addition salts thereof, wherein

$R^1$ is hydrogen, ($C_1$-$C_6$)alkyl, or a pharmaceutically acceptable cation;

$R^2$ is ethyl, fluoroethyl, p-fluorophenyl, p-hydroxyphenyl or cyclopropyl;

$R^3$ is fluorine or hydrogen; and

$R^4$ is

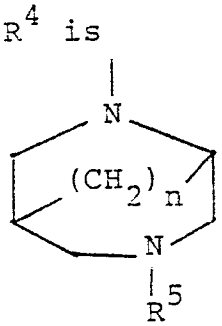

(a)

or

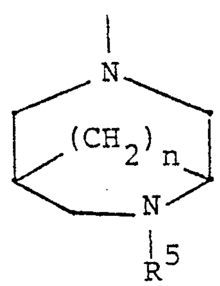

(b)

wherein n is 1 or 2 and $R^5$ is hydrogen or ($C_1$-$C_3$)alkyl.

The present invention also relates to pharmaceutical compositions comprising a pharmaceutically acceptable carrier or diluent and a compound of the formula I or a pharmaceutically acceptable salt thereof in an antibacterially effective amount.

The present invention also provides a method of treating an animal, including a human being, having a bacterial disease which comprises administering to the animal an antibacterially effective amount of a compound of the formula I or a pharmaceutically acceptable salt thereof.

The present invention also relates to methods of preparing the compounds of formula I and to intermediates for the preparation of compounds of the formula I. Such intermediates include compounds of the formulae III, IV, V, VI, VII, VIII, IX, X, XI and XII described below.

The compounds of the present invention may have chiral centers in view of the bridged structures resulting in formation of steroisomers. These steroisomers may be designated with reference to R and S rotation in accordance with standard nomenclature. The compounds of the invention include racemic mixtures and optical isomers.

Preferred compounds of the invention are those of formula I wherein $R^1$ is hydrogen or a pharmaceutically acceptable cation such as sodium or potassium.

Other preferred compounds are those of formula I wherein $R^2$ is ethyl or cyclopropyl and those wherein $R^5$ is methyl or hydrogen. Particularly preferred compounds are those wherein $R^1$ is hydrogen or a pharmaceutically acceptable cation $R^2$ is ethyl or cyclopropyl, and $R^5$ is methyl or hydrogen.

Specific preferred compounds are as follows:

1-cyclopropyl-6-fluoro-1,4-dihydro-7-(6-methyl-3,6-diazabicyclo[3.2.2]non-3-yl)-4-oxo-3-quinolinecarboxylic acid;

1-ethyl-6-fluoro-1,4-dihydro-7-(6-methyl-3,6-diazabicyclo[3.2.2]non-3-yl)-4-oxo-3-quinolinecarboxylic acid;

1-cyclopropyl-6-fluoro-1,4-dihydro-7-(3-methyl-3,6-diazabicyclo[3.2.2]non-6-yl)-4-oxo-3-quinolinecarboxylic acid;

1-ethyl-6-fluoro-1,4-dihydro-7-(3-methyl-3,6-diazabicyclo[3.2.2]non-6-yl)-4-oxo-3-quinolinecarboxylic acid;

1-ethyl-6-fluoro-1,4-dihydro-7-(3-methyl-3,6-diazabicyclo[3.2.1]oct-6-yl)-4-oxo-3-quinolinecarboxylic acid;

1-cyclopropyl-6-fluoro-1,4-dihydro-7-(3-methyl-3,6 diazabicyclo[3.2.1]oct-6-71)-4-oxo-3-quinolinecarboxylic acid;

1-ethyl-6-fluoro-1,4-dihydro-7-(6-methyl-3,6-diazabicyclo[3.2.1]oct-3-yl)-4-oxo-3-quinolinecarboxylic acid; and

1-cyclopropyl-6-fluoro-1,4-dihydro-7-(6-methyl-3,6-diazabicyclo[3.2.1]oct-3-yl)-4-oxo-3-quinolinecarboxylic acid.

The pharmaceutical compositions of the present invention preferably contain the above preferred and specific preferred compounds.

The compounds of formula I may be prepared by reacting a compound of the formula

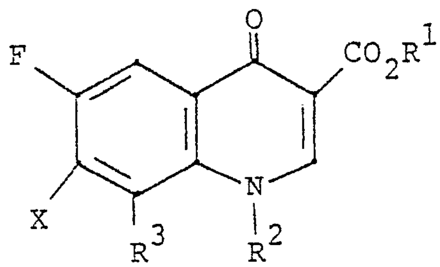

II

with a compound of the formula $R^4H$ wherein $R^1$, $R^2$, $R^3$ and $R^4$ are as defined above in connection with formula I, and X is halogen (e.g., fluoro, chloro or bromo).

The reaction may be performed with or without a solvent, preferably at elevated temperature, and for a time sufficient to substantially complete the reaction. The reaction is preferably carried out in the presence of an acid acceptor such as an inorganic or organic base, e.g., an alkali metal or alkaline earth metal carbonate or bicarbonate or a tertiary amine such as triethylamine, pyridine, picoline, or 1,5-diazabicyclo[5.4.0]undec-5-ene (DBU).

The solvents for this reaction are solvents which are non-reactive under the reaction conditions such as acetonitrile, tetrahydrofuran, ethanol, chloroform, dimethylsulfoxide (DMSO), dimethylformamide, pyridine, water, or mixtures thereof.

The reaction temperature usually ranges from about 20°C to about 150°C.

Starting materials of formula II are known in the art, e.g., as disclosed in European Patent Application 86307045.4 (Publication No. 0215650).

The starting materials of formula $R^4H$ have the following formulae

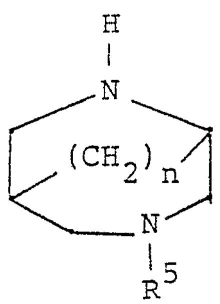

III

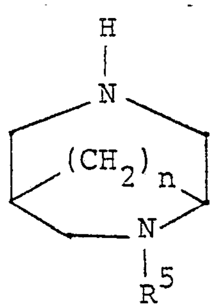

IV

wherein n and $R^5$ are as defined above. The compounds of formulae III and IV are novel compounds. The following reaction schemes illustrate the preparation of these compounds.

Scheme 1

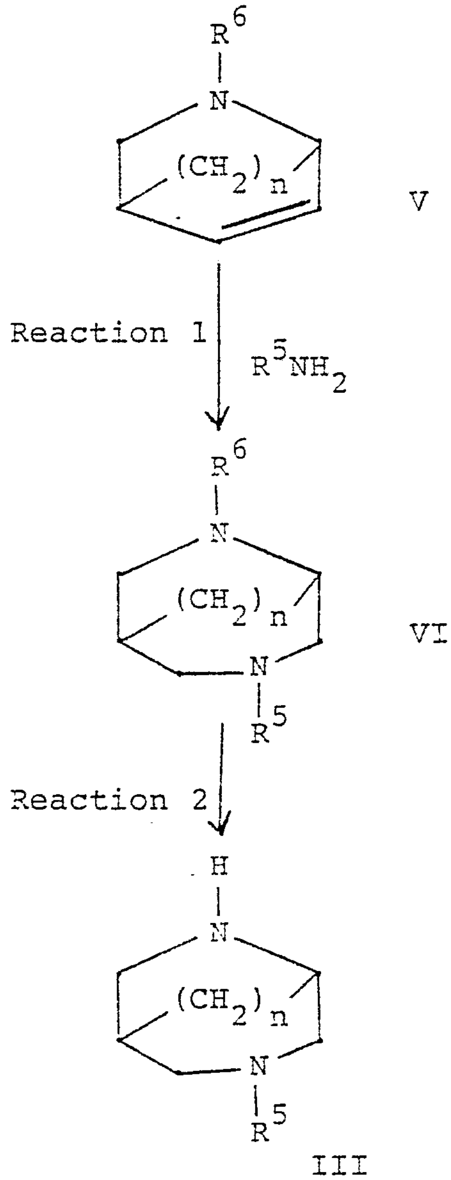


Scheme 2

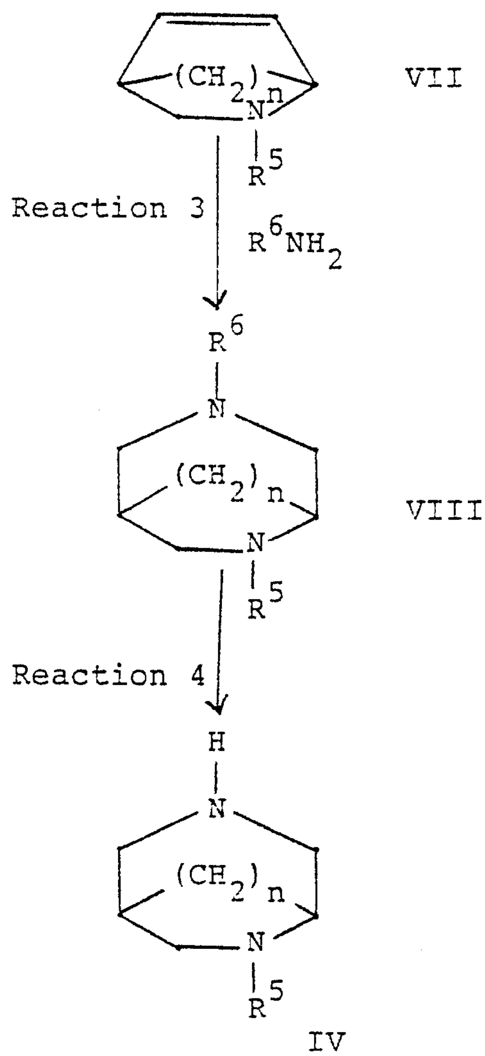

Scheme 3

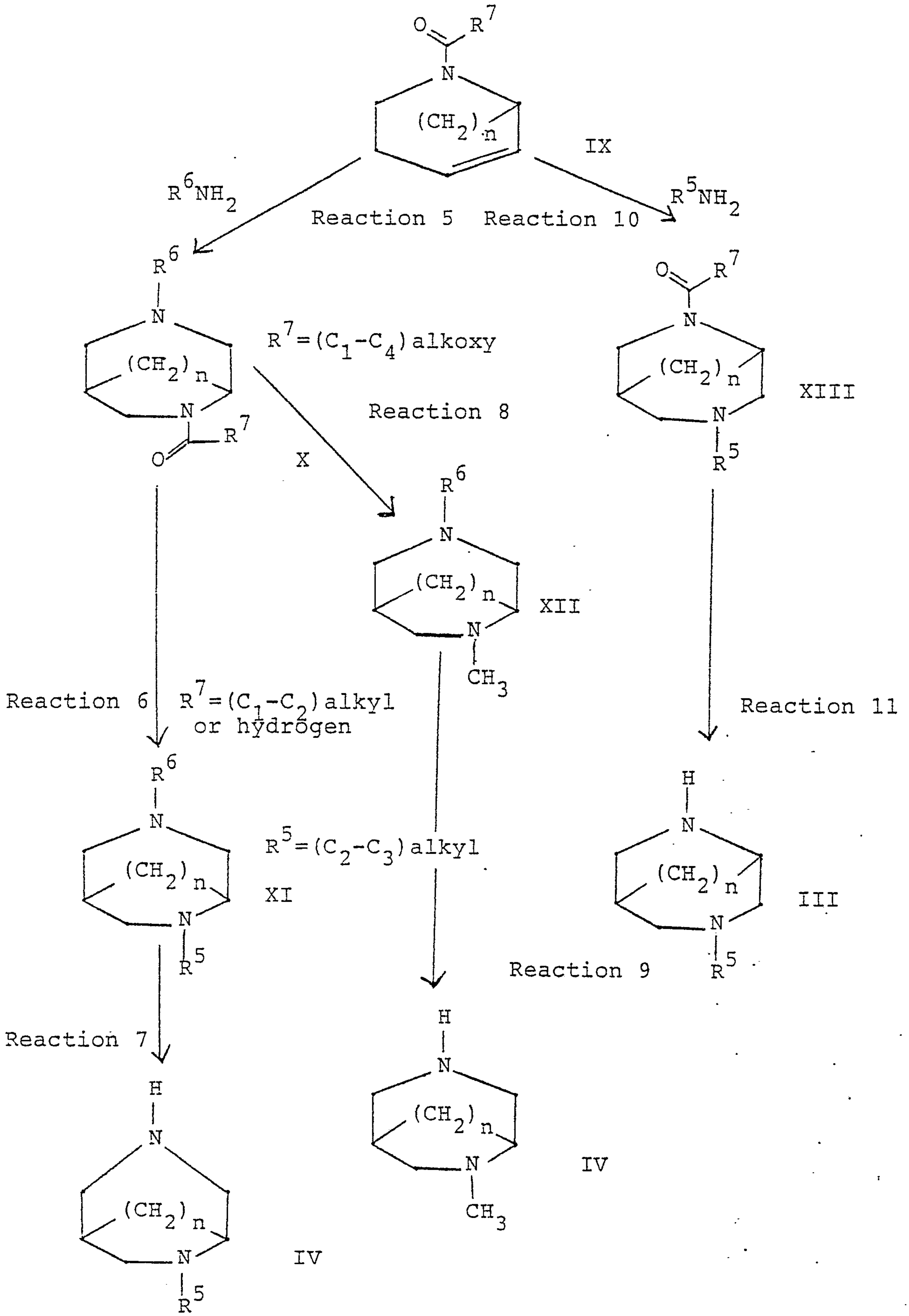
IX
$R^6NH_2$
Reaction 5
Reaction 10
$R^5NH_2$
$R^7=(C_1-C_4)$alkoxy
Reaction 8
X
XIII
XII
Reaction 6
$R^7=(C_1-C_2)$alkyl or hydrogen
Reaction 11
$R^5=(C_2-C_3)$alkyl
XI
III
Reaction 9
Reaction 7
IV
IV

As shown in Schemes 1, 2 and 3, a compound of the formula III may be prepared from a compound of the formula V or a compound of the formula IX and a compound of the formula IV may be prepared from a compound of the formula VII or a compound of the formula IX.

In the foregoing schemes, n is 1 or 2; $R^5$ is as defined above; $R^6$ is hydrogen or a group easily removed under reductive (e.g., hydrogenolysis) conditions (for example, benzyl para-methoxy benzyl or para-nitro benzyl); $R^7$ is hydrogen, $(C_1-C_4)$alkyl or $(C_1-C_4)$alkoxy. Preferred $R^6$ groups are hydrogen and benzyl.

Compounds of the formula V, VII and IX may be prepared by an aza Diels-Alder reaction (see S. Weinreb et al., Heterocycles, 12, 949 (1979)). Preferably, compounds of the formula V and VII are prepared by the method of Grieco (S.D. Larson et al., J.Am. Chem.Soc. 107, 1768 (1985)). Compounds of the formula IX wherein n is 2 are preferably prepared by the method of Cava (M. P. Cava et al., J. Org. Chem., 30, 3772 (1965)). Unless indicated otherwise, the pressures under which the reactions of the above scheme are carried out are not critical but ambient pressure (i.e., about 1 atmosphere) is preferred.

In Reactions 1, 3, 5 and 10, a compound of the formula V, VII, or IX is dissolved in a protic or an aprotic solvent (in the case of reactions 1 and 3, an equivalent of acid (e.g., hydrochloric acid) is also added). The solvent is preferably methanol. The solution is maintained at a temperature of about -78 to about 0°C and is exposed first to an oxidizing agent (preferably, ozone) and then to a reducing agent (preferably, dimethylsulfide). The reaction mixture is then maintained at about -10 to about -25°C and an acid salt (preferably, the hydrochloride salt) of an appropriately substituted amine is added, together with a reducing agent (preferably, sodium cyanoborohydride, to prepare a compound of the formula VI, VIII, X or XIII by reductive amination. Further reactions proceed as follows:

Reaction 11

A compound of the formula XIII is converted to a compound of the formula III by acid hydrolysis, for example, by dissolving the compound of the formula VI in aqueous HBr and refluxing the reaction mixture.

Reactions 6 and 8

A compound of the formula X is converted to a compound of the formula XI (when $R^7$ is $C_1-C_2$) alkyl) or to a compound of the formula XII (when $R^7$ is $(C_1-C_4)$ alkoxy) by reduction in an aprotic solvent at a temperature of about 0 to about 150°C. The solvent is preferably tetrahydrofuran and the reducing agent is preferably lithium aluminum hydride. It should be noted that in Reaction 6, the $COR^7$ group is converted to $R^5$.

Reactions 2, 4, 7 and 9

A compound of the formula VI is converted to a compound of the formula III, a compound of the formula VIII is converted to a compound of the formula IV, a compound of the formula XI is converted to a compound of the formula IV, and a compound of the formula XII is converted to compound of formula IV by reduction in a protic or aprotic solvent at a temperature of about 25 to about 100°C. Preferred solvents are methanol and acetic acid. Preferably, the reduction is accomplished by hydrogenation at a pressure of about 1 to about 100 atmospheres (preferably about 1 to about 5 atmospheres) in the presence of a transition metal catalyst (preferably, $Pd(OH)_2$ or Pd on carbon in the presence of an acid (preferably, HCl). It should be noted that when $R^6$ is hydrogen, Reactions 2, 4, 7, and 9 are not necessary.

The pharmaceutically acceptable acid addition salts of the compounds of the formula I are prepared in a conventional manner by treating a solution or suspension of the free base of the formula I with about one chemical equivalent of a pharmaceutically acceptable acid. Conventional concentration and recrystallization techniques are employed in isolating the salts. Illustrative of suitable acids are acetic, lactic, succinic, maleic, tartaric, citric, gluconic, ascorbic, benzoic, methanesulfonic, cinnamic, fumaric, phosphonic, hydrochloric, hydrobromic, hydroiodic, sulfamic and sulfonic acid.

The pharmaceutically acceptable cationic salts of the compounds of the formula I may be prepared by conventional methods form the corresponding acids, e.g., by reaction with about one equimolar amount of a base. Examples of suitable cationic salts are those of alkali metals such as sodium or potassium, alkaline earth metals such as magnesium or calcium, and ammonium or organic amines such as diethanol amine or N-methylglucamine.

The compounds of formula I and the pharmaceutically acceptable acid addition salts thereof are useful in the treatment of bacterial infections of broad spectrum, particularly the treatment of infections of gram-positive bacterial strains.

The compounds of the present invention may be administered alone, but will generally be administered in admixture with a pharmaceutical carrier selected with regard to the intended route of administration and standard pharmaceutical practice. For example, they can be administered orally or in the form of tablets containing such excipients as starch or lactose, or in capsules either alone or in admixture with excipients, or in the form of elixirs or suspensions containing flavoring or coloring agents. In the case of animals, they are advantageously contained in an animal feed or drinking water in a concentration of about 5 to about 5000 ppm, preferably about 25 to about 500 ppm. They can be injected parenterally, for example, intramuscularly, intravenously or subcutaneously. For parenteral administration, they are best used in the form of a sterile

aqueous solution which can contain other solutes, for example, enough salt or glucose to make the solution isotonic. In the case of animals, compounds can be administered intramuscularly or subcutaneously at dosage levels of about 0.1 to about 50 mg/kg/day, advantageously about 0.2 to about 10 mg/kg/day given in a single daily dose or up to 3 divided doses.

The invention also provides pharmaceutical compositions comprising an antibacterially effective amount of a compound of the formula I or a pharmaceutically acceptable acid addition salt thereof together with a pharmaceutically acceptable diluent or carrier.

The compounds of the present invention can be administered to humans for the treatment of bacterial diseases by either the oral or parenteral routes, and may be administered orally at dosage levels of about 0.1 to about 500 mg/kg/day, advantageously about 0.5 to about 50 mg/kg/day given in a single dose or up to 3 divided doses. For intramuscular or intravenous administration, dosage levels are about 0.1 to about 200 mg/kg/day, advantageously about 0.5 to about 50 mg/kg/day. While intramuscularly administration may be a single dose or up to 3 divided doses, intravenous administration can include a continuous drip. Variations will necessarily occur depending on the weight and condition of the subject being treated and the particular route of administration chosen as will be known to those skilled in the art.

The antibacterial activity of the compounds of the invention is shown by testing according to the Steer's replicator technique which is a standard in vitro bacterial testing method described by E. Steers et al., Antibiotics and Chemotherapy, 9, 307 (1959).

The following non-limiting examples illustrate the invention. All melting points referred to in the Examples are uncorrected.

## Example 1

### 2-Carboethoxy-2-azabicyclo[2.2.2]oct-5-ene

Following the procedure of Cava (M. P. Cava et al., J. Org. Chem., 30, 3772 (1965)), with the Borne modification(R. F. Borne et al., J. Med. Chem., 16, 853 (1973)), 50.0 g (0.32 mol) of methylene bisurethane and 31 ml (26.1 g, 0.33 mol) of 1,3-cyclohexadiene were condensed in 415 ml of benzene in the presence of 8.7 ml (10.0 g) of $BF_3$ etherate to provide 25.0 g (43%) of the title compound as a clear liquid, bp 74-75°C/0.2 Torr.

## Example 2

### 3-Benzyl-6-carboethoxy-3,6-diazabicyclo[3.2.2]nonane

A stream of ozone was passed through a 1.2 L methanol solution of 40.0 g (0.22 mol) of 2-carboethoxy-2-azabicyclo[2.2.2]oct-5-ene at -78°C for 5.5 hours. At that time, the reaction mixture became blue and it was found by thin layer chromotography (TLC) on silica gel, using 1:1 EtOAc(ethyl acetate)/hexane as the eluant, that the starting olefin had been consumed. Nitrogen was bubbled through the reaction mixture to remove excess ozone and 100 ml of dimethylsulfide was then added dropwise. The reaction mixture was then allowed to warm slowly to 0°C. Following 4 hours of additional stirring at 0°C, 200 g (1.39 mol) of benzylamine hydrochloride and 200 g of activated 3Å molecular sieves were added and the reaction mixture was stirred for 4 hours at room temperature. The reaction mixture was then cooled to 0°C and treated with a 200 ml methanol solution of 69.8 g (1.11 mol) of sodium cyanoborohydride dropwise over 40 minutes. Additional stirring was continued for 5 hours at room temperature followed by filtration, as the insoluble material was washed well with methanol. The filtrate was concentrated to an oil, cooled to 0°C, and carefully acidified (HCN evolution) with 500 ml of aqueous 1N HCl with stirring. After the effervescence had ceased (4 hours), sodium hydroxide pellets were added carefully at 0°C until the mixture was basic (pH 13). The oil that had separated was removed in a separatory funnel and combined with 5 x 100 ml EtOAc extracts of the aqueous layer which were then dried ($K_2CO_3$) and evaporated. Partial distillation of the oil (60°C, 2 Torr} to remove excess benzylamine and chromotography of the residue (63 g) on 290 g of silica gel using an EtOAc-hexane eluant (1:9 to 3:7) afforded 22.2 g of the title compound ($R_f$ 0.65, EtOAc-hexane, 1:1) as an oil. The product was further purified by short-path distillation to provide 21.1 g (33%) as a pale yellow oil, bp 166-168°C/0.1 Torr: $^1$H-NMR ($CDCl_3$, 250 MHz) δ 1.20 and 1.24 (3H, two t, J=7), 1.4-1.6 (1H, m), 3.55 and 3.56 (3H, two S), 4.12 and 4.13 (2H, two quartets, J=7), 7.2-7.4 (5H, m); IR($CHCl_3$) 1673 $cm^{-1}$ ; MS (m/e) 288 ($M^+$), 245, 197, 134, 91.

Anal. Calcd.. for $C_{17}H_{24}N_2O_2$ : C, 70.80; H, 8.39; N, 9.71. Found: C, 70.41; H, 8.20; N, 9.66.

## Example 3

### 3-Benzyl-6-methyl-3,6-diazabicyclo[3.2.2]nonane

To a suspension of 6.32 g (0.167 mol) of lithium aluminum hydide in 1.5 L of ether was added dropwise over 45 minutes a 200 ml ether solution of 20.96 g (0.073 mol) of 3-benzyl-6-carboethoxy-3,6-diazabicyclo[3.2.2]nonane. After a gentle reflux period of 2 hours, the mixture was chilled to 0° C and carefully quenched by the sequential addition of 6.32 ml of water, 6.32 ml of aqueous 15% aqueous NaOH solution, and 19.0 ml of water. The resulting salts were removed by filtration and were washed well with ether. The filtrate was dried ($K_2CO_3$) and evaporated to a clear oil which was distilled to provide 15.13 g (90%) of the title compound as a clear liquid, bp 120-122° C/0.1 Torr: $^1H$-NMR ($CDCl_3$, 250 MHz) δ 1.5-1.7 (1H, m), 1.8-2.0 (m, 3H), 2.4 (3H, S), 3.55 (2H, centroid of AB pattern, J = 15), 7.2-7.4 (5H, m); $^{13}$ C-NMR ($CDCl_3$, 63 MHz)δ 22.7, 24.7, 31.8, 44.1, 57.2, 57.4, 58.1, 62.1, 62.5, 126.8, 128.2, 140.0.

Anal. Calcd. for $C_{17}H_{24}N_2O_2$; C, 78.21; H, 9.63; N, 12.16. Found: C, 78.42; H, 9.59; N, 12.08.

## Example 4

### 6-Methyl-3,6-diazabicyclo[3.2.2]nonane Dihydrobromide

A 330 ml 1N HCl solution of 15.13 g (0.066 mol) of 3-benzyl-6-methyl-3,6-diazabicyclo[3.2.2]nonane was hydrogenated at 30 psi in the presence of 2.6 g of 10% Pd/C for 3 hours. After filtration of the catalyst and concentration of the filtrate, an oily residue was obtained. The oily residue was stirred for 2 hours in a 1 L methanol suspension of 250 g of Amberlite CG-400II ion-exchange resin (Fluka Chemical Corporation) which was prewashed sequentially with water, aqueous 1N NaOH solution, water, and methanol. The resin was then filtered and washed well with methanol, and the filtrate was acidified with 140 ml of aqueous 48% HBr solution. The solvent was removed and the residue was crystallized from isopropanol to provide 14.89 g (75%) of the title compound as a white solid, mp 295-300° C (dec.): $^1H$-NMR ($D_2O$, 300 MHz) δ 1.95-2.25 (3H, m), 2.35-2.54 (1H, m), 2.60-2.72 (1H, m), 2.03 (3H, S), 3.40-4.07 (7H, m); $^{13}C$-NMR ($D_2O$, 75 MHz) δ 18.3, 28.0, 43.3, 52.5, 56.9, 57.5.

Anal. Calcd.. for $C_8H_8N_2Br_2$: C, 31.81; H, 6.01; N, 9.27; Br, 52.91. Found: C, 31.72; H, 5.89; N, 9.23.

## Example 5

### 1-Cyclopropyl-6-fluoro-1,4-dihydro-7-(6-methyl-3,6-dia-zabicyclo[3.2.2] non-3-yl)-4-oxo-3-quinolinecarboxylic Acid

A mixture of 1.00 g (3.77 mmol) of 1-cyclopropyl-6,7-difluoro-1,4-dihydro-4-oxo-3-quinolinecarboxylic acid, 2.51 g (8.30 mmol) of 6-methyl-3, 6-diazabicyclo[3.2.2]nonane dihydrobromide, 2.89 ml (2.82 g, 18.85 mmol) of 1,5-diazabicyclo[5.4.0]undec-5-ene (DBU), and 100 ml of pyridine was heated to 80°C for 4.5 hours. The solvent was removed and the yellow residue was triturated with 60 ml of isopropanol and then washed with 4.0 ml of ether to provide 0.93 g of a pale yellow solid, mp 215-220° C. Recrystallization from methanol afforded 546 mg (38%) of the title compound, mp 225-226.5° C: $^1H$-NMR ($CDCl_3$ and $CD_3OD$, 250 MHz) δ 1.6-2.2 (5H, m), 2.25 (1H, broad S), 2.44 (3H, S), 2.80-3.02 (3H, m), 3.34-3.60 (4H, m), 3.70 (1H, dd, J-5.12), 7.32 (1H, d, J = 6), 7.97 (1H, d, J = 13), 8.72 (1H, S).

Anal. Calcd.. for $C_{21}H_{24}FN_3O_3 \cdot \frac{1}{4}H_2O$: C, 64.58; H, 6.33; N, 10.78. Found: C, 64.63; H, 6.13; N, 10.73.

## Example 6

### 1-Ethyl-6-fluoro-1,4-dihydro-7-(6-methyl-3,6-diazabicyclo[3.2.2]non-3-yl)-4-oxo-3-quinolinecarboxylic Acid

A mixture of 84.5 mg (0.334 mmol) of 1-ethyl-6-fluoro-1,4-dihydro-7-4-oxo-3-quinolinecarboxylic acid (see British Patent 2,093,018), 117 mg (0.834 mmol) of 6-methyl-3,6-diazabicyclo[3.2.2]nonane (prepared by catalytic hydrogenation of 3-benzyl-6-methyl-3,6-diazabicyclo[3.2.2]nonane over $Pd(OH)_2$), and 5 ml of

pyridine was heated to 80° C for 12 hours. The solvent was evaporated and the residue was purified by silica gel chromatography (4 cm diameter, 2 cm height) using 15% $CH_3OH/CHCl_3$ as eluant to give 84 mg of a yellow solid. Trituration in ether afforded 39 mg of the title compound as a pale yellow powder, $R_f$ 0.1, 15% $CH_3OH/CHCl_3$), mp 175-177° C; $^1H$-NMR (250 MHZ, $CDCl_3$) δ 1.56 (3H, t, J=7), 2.52 (3H, S), 2.92 (2H, dd, J=4,14), 3.00 (2H, d, J=12), 3.43 (1H, d, J=12), 3.52 (2H, d, J=13), 3.74 (1H, dd, J=7,12), 4.32 (2H, q, J=7), 6.82 (1H, d, J=7), 8.04 (1H, d, J=12), 8.67 (1H, S). HRMS, found 373.1724, calcd. for $C_{20}H_{24}FN_3O_3$, 373.1799.

## Example 7

### 6-Carboethoxy-3-methyl-3,6-diazabicyclo[3.2.2]nonane

Following the procedure for the synthesis of 3-benzyl-6-carboethoxy-3,6-diazabicyclo[3.2.2]nonane described in Example 2, 10 g (.055 mol) of 2-carboethoxy-2-azabicyclo[2.2.2]oct-5-ene was ozonolyzed and subjected to reductive amination using 37.3 g (0.552 mol) of methylamine hydrochloride and 17.3 g (0.276 mole) of sodium cyanoborohydride in the presence of 38 g of activated 3A° molecular sieves. After work-up, the reaction mixture (13.2 g) was purified by silica gel chromatography (10 cm height, 11.5 cm diameter) using 5% $CH_3OH/CHCl_3$ as eluant to provide 3.89 g (33%) of the title compound as a pale yellow oil ($R_f$ 0.31, 5% $CH_3OH/CHCl_3$) : $^1H$-NMR (250 MHZ, $CDCl_3$) δ 1.15 and 1.18 (3H, two t, J=7), 1.37-1.50 (1H, m), 2.205 and 2.21 (3H, two S), 2.73-2.93 (2H, m), 3.15-3.27 (1H, m) 4.05 (2H, q, J=7); IR($CHCl_3$) 1672 $cm^{-1}$; HRMS, found 212.1523 ($M^+$), calcd. for $C_{11}H_{20}N_2O_2$ 212.1524.

## Example 8

### 3-Methyl-3,6-diazabicyclo[3.2.2]nonane Dihydrobromide

A mixture of 852 mg of 6-carboethoxy-3-methyl-3,6-diazabicyclo[3.2.2]nonane and 15 ml of 33% HBr solution in acetic acid was heated to 70° C for 4 hours. Evaporation of the solvent and trituration of the solid residue in isopropanol afforded 951 mg (78%) of the title compound as a fluffy, tan solid, mp 297-300° C: $^1H$-NMR (250 MHZ, $D_2O$) δ 2.02 (4H, m), 2.58-2.68 (1H, m), 3.04 (3H, S), 3.45-3.75 (4H, m), 3.85-4.15 (3H, m) ; $^{13}C$-NMR(63 MHZ, $D_2O$) 18.5, 19.6, 27.2, 45.1, 45.9, 48.0, 58.4, 62.7.

Anal. Calcd. for $C_8H_{18}N_2BR_2$ ; C, 31.81; H, 6.01; N, 9.27. Found: C, 31.46; H, 5.82; N, 9.11.

## Example 9

### 1-Cyclopropyl-6-fluoro-1,4-dihydro-7-(3-methyl-3,6-diazabicyclo[3.2.2]non-6-yl)-4-oxo-3-quinolinecarboxylic Acid

A mixture of 57 mg (0.216 mmol) of 1-cyclopropyl-6,7-difluoro-1,4-dihydro-4-oxo-3-quinolinecarboxylic acid, 163 mg (0.54 mmol of 3-methyl-3,6-diazabicyclo[3.2.2]nonane dihydrobromide, 177 μl(181 mg, 1.19 mmol) of 1,5-diazabicyclo[5.4.0]undec-5-ene(DBU), and 100 ml of pyridine was heated to 80° C for 4 hours. The solvent was evaporated and the residue crystallized from isopropanol to afford 48 mg of the title compound as a white solid ($R_f$ 0.14, 1BuOH (butyl alcohol), $1H_2O$, $CH_3COOH$, 1EtOAc), mp 253-256° C: HRMS, found 385.1814, calcd. for $C_{21}H_{24}O_3N_3F$ 385.1782.

## Example 10

### 1-Ethyl-6-fluoro-1,4-dihydro-7-(3-methyl-3,6-diazabicyclo[3.2.2]non-6-yl)-4-oxo-3-quinolinecarboxylic Acid

A mixture of 7.4 mg (0.0291 mmol) of 1-ethyl-6,7-difluoro-1,4-dihydro-4-oxo-3-quinolinecarboxylic acid (British Patent 2093018), 22 mg (0.0728 mmol) of 3-methyl-3,6-diazabicyclo[3.2.2]nonane dihydrobromide, 22 mg (0.146 mmol) of 1,5-diazabicyclo[5.4.0]undec-5-ene, and 2 ml of pyridine was maintained at 100° C for 16 hours. The solvent was evaporated and the residue was diluted with $CHCl_3$ and extracted with an aqueous 1N HCl solution. The acidic extracts were backwashed with fresh $CHCl_3$, adjusted to pH8 with saturated aqueous

$NaHCO_3$ solution, and extracted with $CHCl_3$. Drying ($Na_2SO_4$) of the $CHCl_3$ extracts and evaporation gave 17 mg of a yellow solid which was triturated in ether providing 10 mg of a solid, mp 177-185° C. Purification of the solid by silica gel chromatography (2 cm diameter, 8 cm height} with a 10% $CH_3OH/CHCl_3$ eluant gave 7 mg (64%} of the title compound mp 198-203° C: $^1$H-NMR (250 MHZ, $CDCl_3$) δ 1.58 (2H, t, J=6), 2.37 (3H, S), 2.46 (1H, d, J=12), 2.64 (1H, d, J=12), 4.25 (2H, q, J=8), 6.48 (1H, d, J=7), 7.96 (1H, d, J=12), 8.58 (1H, S); HRMS, found 373.1852, calcd. for $C_{20}H_{24}FN_3O_3$ 373.1799.

## Example 11

### 2-Methyl-2-azabicyclo[2.2.1]hept-5-ene

Following the procedure of Grieco (S. D. Larson et al., J. Am. Chem. Soc. 107, 1768 (1985)), a mixture of 7.11 g (0.105 mol) of methylamine hydrochloride, 11.9 g (0.147 mol) of aqueous 37% formaldehyde solution, 13.9 g (0.211 mol) of cyclopentadiene, and 30 ml of water was stirred vigorously for 16 hours at room temperature. The mixture was diluted with 50 ml of water and the separated oil was extracted with ether (4 x 30 ml). The aqueous layer was basified by the addition of 6 g of KOH pellets and the mixture was extracted with ether (4 x 30 ml). Drying of the combined extracts ($K_2CO_3$) and evaporation of the solvent afforded 6.29 g (55%) of the title compound as an oil; $^1$H-NMR ($CDCl_3$, 90MHz δ 1.3-1.7 (3H, m), 2.19 (3H, S), 2.94 (1H, broad S), 2.21 (1H, dd, J=9 and 3), 3.80 (1H, broad S), 6.0-6.5 (2H, m).

## Example 12

### 3-benzyl-6-methyl-3,6-diazabicyclo[3.2.1]octane

A solution of 5.07 g (46.4 mmol) of 2-methyl-2-azabicyclo[2.2.l]hept-5-ene in 30 ml of dioxane was treated with 20 ml of a 4.5N solution of hydrogen chloride in dioxane. After removal of the solvent, the residue was dissolved in 100 ml of methanol and the mixture was ozonolyzed at -78° C until a blue color persisted. 14 ml (11.8 g, 190 mmol) of dimethylsulfide was then added and the mixture was allowed to warm slowly to 0° C. The mixture was transferred to a 3-neck flask equipped with a mechanical stirring apparatus and 66.6 g (464 mmol) of benzylamine hydrochloride and 50 g of activated 3A molecular sieves were added at 0° C. The suspension was stirred for 16 hours as the ice bath was allowed to melt and then rechilled to 0° C before being treated with 14.6 g (232 mmol) of solid sodium cyanoborohydride. Following slow warming to room temperature over a 4.5-hour period, the insoluble material was removed by filtration and the filtrate was evaporated. The residue was diluted with 150 ml of water and carefully acidified by the dropwise addition of 200 ml of aqueous 6N HCl solution (HCN evolution) and the resulting mixture was stirred 16 hours at room temperature before being carefully basified (pH 14) with NaOH pellets. The mixture was extracted with ethyl acetate (4 x 200 ml) and the extracts were dried ($K_2CO_3$), evaporated, and the excess benzylamine was removed by partial fractional distillation under high vacuum. Purification of the undistilled residue by silica gel chromatography (7 cm diameter, 16 cm height) using 89:$CHCl_3$, 10:$CH_3OH$, 1: concentrated aqueous $NH_4OH$ (proportions by volume) as eluant and then by distillation (bp 105-106°C/O.5 Torr) afforded 4.00 g (40%) of 3-benzyl-6-methyl-3,6-diazabicyclo[3.2.1]octane as a pale yellow oil ($R_f$ 0.1, 89:$CHCl_3$, 10:MeOH, 1:concentrated aqueous $NH_4OH$): $^1$H-NMR ($CDCl_3$, 250 MHz) δ 1.44 (1H, d, J=10), 1.9-2.0 (1H, m), 2.10 (2H, d, J=8), 2.2-2.3 (1H, m), 2.41 (3H, S), 2.70 (2H, d, J=10), 2.9-3.1 (3H, m), 3.50 (2H, centroid of AB quartet, J=12), 7.15-7.35 (5H, m); $^{13}$C-NMR ($CDCl_3$, 63 MHz) δ 35.4, 36.8, 37.7, 54.9, 57.7, 58.3, 58.7, 62.3, 126.8, 128.0, 128.8, 138.8; HRMS, found 216.1628, calcd. for $C_{14}H_{20}O_2$ 216.1641.

## Example 13

### 6-Methyl-3,6-diazabicyclo[3.2.1]octane

A solution of 509 mg (2.35 mmol) of a 3-benzyl-6-methyl-3,6-diazabicyclo[3.2.1]octane in 5 ml of methanol was hydrogenated at 45 psi for 10 hours in the presence of 500 mg of $Pd(OH)_2$ (Pearlman's catalyst). The catalyst was removed by filtration and the solvent was carefully evaporated to 462 mg (greater than theory) of the title compound as an oil: $^1$H-NMR ($CDCl_3$, 60 MHz) δ 1.7 (1H, d, J=10), 2.8 (3H, S); MS (m/e), 216 ($M^+$).

## Example 14

### 6-Methyl-3,6-diazabicyclo[3.2.1]octane Dihydrochloride

A mixture of 3.00 g of 3-benzyl-6-methyl-3,6-diazabicyclo[3.2.l]octane, 75 ml of methanol and 5 ml of concentrated aqueous hydrochloric acid was hydrogenated at 50 psi in the presence of 1 g of 10% Pd/C for 7 hours. The catalyst was removed by filtration and the filtrate was evaporated to a solid which was triturated in isopropanol and ether to afford 2.43 g (88%) of 6-methyl-3,6-diazabicyclo[3.2.1]octane dihydrochloride, mp 266-269°C.: $^{1}$H-NMR ($D_2O$, 300 MHz) δ 2.22 (1H, d, J=13), 2.3-2.5 (1H, m), 3.06 (3H, s), 3.45 (1H, s), 3.53 (1H, d, J=13), 3.77 (1H, d, J=13), 4.18 (1H, s); $^{13}$C-NMR ($D_2O$ + NaOD, 63 MHz) 35.1, 37.0, 41.3, 48.4, 50.4, 58.3, 61.0.

## Example 15

### 1-Ethyl-6-fluoro-1,4-dihydro-7-(6-methyl-3,6-diazabicyclo[3.2.1]oct-3-yl)-4-oxo-3-quinolinecarboxylic Acid

A mixture of 297 mg (2.35 mmol of 6-methyl-3,6-diazabicyclo[3.2.1]octane, 228 mg (0.94 mmol) of 1-ethyl-6,7-difluoro-1,4-dihydro-4-oxo-3-quinolinecarboxylic acid and 10 ml of pyridine was heated to reflux and maintained at reflux for 16 hours. The solvent was evaporated and the residue was diluted with $CHCl_3$ and repeatedly extracted with aqueous 1N HCl solution. The combined acidic extracts were backwashed once with $CHCl_3$ and the pH was adjusted to 8.5 with aqueous NaOH solution. Extraction of this mixture with $CHCl_3$, drying ($Na_2SO_4$), and evaporation gave 441 mg of a brown solid which was further purified by silica gel chromatography (4 cm diameter, 2 cm height) using 18%$CHCl_3$, 2%$CH_3OH$, O.5%$CH_3COOH$ (all percents by volume as eluant. Evaporation of the appropriate fractions containing the fluorescent product and acid-base work-up (as described above) of the residue to remove acetic acid gave a yellow solid which was triturated in ether to 15 mg (4%) of the title compound as a yellow powder, mp 175-180°C; $^{1}$H-NMR (DMSO $d^6$, 250 MHz δ 1.44 (3H, t, J=7), 1.7 (1H, d, J=10), 1.85-2.0 (1H, m), 1.42 (3H, S), 2.63 (1H, d, J=7), 3.87 (1H, d, J=11), 4.59 (2H, broad q), 7.13 (1H, d, J=7), 7.90 (1H, d, J=13), 8.94 (1H, S).

## Example 16

### 1-Cyclopropyl-6-fluoro-1,4-dihydro-7-(6-methyl-3,6-diazabicyclo[3.2.l]oct-3-yl)-4-oxo-3-quinolinecarboxylic Acid

A mixture of 212 mg (0.80 mmol) of 1-cyclopropyl-6,7-difluoro-1,4-dihydro-l-oxo-3-quinolinecarboxylic acid, 400 mg (2.00 mmol) of 6-methyl-3,6-diazabicyclo[3.2.1]octane dihydrochloride, 487 mg (3.20 mmol) of 1,5-diazabicyclo[5.4.0]undec-5-ene (DBU), and 10 ml of pyridine was stirred at 60°C for 16 hours. The solvent was removed by rotary evaporation and the residue was purified by flash chromatography on silica gel (2.5 in height x 30 mm diameter) using 95:5:1, $CHCl_3$:$CH_3OH$: acetic acid as eluant gave 123 mg of a white solid. Trituration in ether afforded 41 mg (13%) of the title compound, mp 226-228°C (dec). $^{1}$H-NMR (300 MHZ, $CDCl_3$) δ 1.0-1.2 (4H, m), 1.66 (1H, d, J=12), 1.95-2.0 (1H, m), 2.0-2.05 (1H, m), 2.50 (3H, s), 3.67 (1H, broad d), 3.84 (1H, broad d), 7.22 (1H, broad d), 7.84 (1H, d, J=13), 8.59 (1H, s); MS m/e 371($M^+$), 351, 290, 82 (base).

## Example 17

### 2-Benzyl-2-azabicyclo[2.2.1]hept-5-ene

Following the procedure of Grieco, (S. D. Larson et al., J. Am. Chem. Soc., 107, 1768 (1985)), a mixture of 5.0 g (0.0348 mol) of benzylamine hydrochloride, 3.95 g (0.0487 mol) of aqueous 37% formaldehyde solution, 4.6 g (0.0696 mol) of cyclopentadiene, and 10 ml of water was stirred vigorously for 16 hours at room temperature. The mixture was diluted with 20 ml of water and the separated oil was extracted with ether (2 x 20 ml). The aqueous layer was basified by the addition of 4 g of KOH pellets, and the turbid mixture was extracted with ether (3 x 25 ml). Drying ($K_2CO_3$) of the combined extracts and evaporation of the solvent afforded 7.5 g

(greater than theory) of the title compound as an oil: $^1$H-NMR(90 MHz, $CDCl_3$) δ 1.3-1.8 (3H, m), 2.95 (1H, S), 3.3-3.1 (1H, m), 3.49 (2H, centroid of AB pattern, J = 14), 3.85 (1H, S), 6.05-6.2 (1H, m), 6.35-6.5 (1H, m), 7.25-7.5 (5H, m).

## Example 18

### 6-Benzyl-3-methyl-3,6-diazabicyclo[3.2.l].octane

Following the procedure of Example 12, 8.44 g (45.6 mmol) of N-benzyl-2-azabicyclo[2.2.1]hept-5-ene was ozonolyzed as its hydrochloride salt and, following reduction of the ozonide, the crude dialdehyde was subjected to reductive amination using 30.8 g (456 mmol) of methylamine hydrochloride and 14.3 g (228 mmol) of sodium cyanoborohydride. After work-up, the crude product was purified by silica gel chromatography (7 cm diameter, 15 cm height) using 94:5:1 to 89:10:1 (proportions by volume) $CHCl_3$:$CH_3OH$:concentrated aqueous $NH_3$ as eluant and then by distillation (bp 104-107°C/0.5 Torr) to provide 1.77 g (18%) of 6-Benzyl-3-methyl-3,6-diazabicyclo[3.2.1]octane ($R_f$ 0.15, 89:$CHCl_3$, 10:MeOH, 1:concentrated aqueous $NH_3$) as a pale yellow oil: $^1$H-NMR (250 MHz, $CDCl_3$) δ 1.34 (1H, d, J = 9), 1.92 (2H, d, J = 11), 2.10 (1H, d, J = 9), 2.28 (3H, S), 3.88 (2H, centroid of AB quartet, J = 13), 7.1-7.7 (5H, m); $^{13}$C-NMR ($CDCl_3$, 63 MHz) δ 35.0, 35.4, 45.4, 57.0, 57.1, 58.4, 60.8, 126.6, 128.1, 128.5, 140.0; HRMS, found 216.1588, calcd. for $C_{14}H_{20}N_2$ 216.1626.

## Example 19

### 3-Methyl-3,6-diazabicyclo[3.2.1]octane

To a solution of 500 mg (2.35 mmol) of 6-benzyl-3-methyl-3,6-diazabicyclo[3.2.1.]octane in 5 ml of methanol was added 500 mg of palladium hydroxide (Pearlman's catalyst) and the mixture was hydrogenated at 50 psi for 6 hours using a Parr shaker apparatus. The catalyst was removed by filtration and the filtrate was concentrated to 297 mg (100%) of the title compound as a yellow oil: $^1$H-NMR ($CDCl_3$, 300 MHz) δ 1.60 (1H, d, J = 12), 1.9-2.1 (3H, m), 2.28 (3H, s), 2.48 (1H, d, J = 3), 2.6-2.8 (1H, m), 2.2-2.35 (2H, m), 3.42 (1H, d, J = 12), 3.97 (1H, t, J = 4); $^{13}$C-NMR ($CDCl_3$, 75 MHz) δ 34.1, 34.6, 44.5, 48.5, 54.9, 56.6, 59.0.

## Example 20

### 3-Methyl-3,6-diazabicyclo[3.2.1]octane Dihydrochloride

Following the procedure of Example 14, 1.01 g of 6-benzyl-3-methyl-3,6-diazabicyclo[3.2.1]octane was hydrogenated to provide 0.78 g (84%) of 3-methyl-3,6-diazabicyclo[3.2.1] octane dihydrochloride, mp 259-261°C: $^1$H-NMR ($D_2O$, 250 MHz) 2.05-2.2 (1H, m), 2.28 (1H, d, J = 13), 2.98 (3H, s), 3.04 (1H, s), 3.35-3.8 (5H, m), 3.88 (1H, d, J = 13), 4.38 (1H, s); $^{13}$C-NMR ($D_2O$, 63 MHz) δ 33.0, 33.6, 44.8, 48.2, 55.0, 55.5, 58.6. Anal. Calcd. for $C_7H_{14}N_4 \cdot 2HCl \cdot 0.5H_2O$: C,40.40; H,8.23; N, 13.46. Found: C,40.20, H, 7.89; N, 13.32.

## Example 21

### 3-Methyl-3,6-diazabicyclo[3.2.1]octane Dihydrobromide

To a methanolic (150 ml) suspension of 35 g of Amberlite CG-400-II ion exchange resin (Fluka Chemical Corporation), which was prewashed successively with $H_2O$, 1N aqueous NaOH solution, $H_2O$, and methanol, was added 1.79 g (9.00 mmol) of 3-methyl-3,6-diazabicyclo[3.2.1]octane dihydrochloride and the mixture was stirred for 1.5 hours. The resin was filtered off (washed with methanol} and the filtrate was acidified with 30 ml of 48% aqueous HBr solution. The solvent was removed by rotary evaporation and the residue was crystallized from isopropanol to yield 1.42 g (54%) of 3-methyl-3,6-diazabicyclo[3.2.l]octane dihydrobromide as a partially hygroscopic solid, mp 280-283°C. The $^1$H-NMR and $^{13}$C-NMR were essentially identical to that of the corresponding dihydrochloride salt.

Example 22

1-Ethyl-6-fluoro-1,4-dihydro-7-(3-methyl-3,6-diazabicyclo[3.2.l]oct-6-yl)-4-oxo-3-quinolinecarboxylic Acid

A mixture of 235 mg (0.926 mmol) of 1-ethyl-6,7-difluoro-1,4-dihydro-4-oxo-3-quinolinecarboxylic acid, 292 mg (2.31 mmol) of 3-methyl-3,6-diazabicyclo[3.2.1]-octane, and 10 ml of pyridine was maintained at 80° C for 16 hours. The solid residue after evaporation of the solvent was diluted with 20 ml of $CHCl_3$ and extracted with an aqueous 1N HCl solution (3 x 20 ml). The acidic extracts were basified to pH 8 with NaOH solution, saturated with NaCl, and extracted with $CHCl_3$. The $CHCl_3$ extracts were dried ($Na_2SO_4$) and evaporated to 125 mg of a red solid. Purification of this solid by silica gel chromatography (2 cm diameter, 4.5 cm height) with a 10% $CH_3OH/CHCl_3$ eluant gave 29 mg of a yellow solid which was triturated with ether to provide 23 mg (7%) of the title compound, 219-220° C; $^1H$-NMR ($CDCl_3$, 250 MHz) δ 1.56 (3H, t, J=7), 1.74 (1H, d, J=12), 2.0-2.6 (3H, m), 2.28 (3H, S), 1.55-1.65 (1H, m), 2.93 (1H, d, J=12), 3.16 (1H, d, J=12), 3.4-3.7 (2H, m), 4.36 (2H, q, J=7), 4.45-4.6 (1H, m), 6.39 (1H, d, J=5), 7.95 (1H, d, J=13), 8.56 (1H, S), HRMS, found 359.1643, calcd. for $C_{19}H_{22}FN_3O_3$ 359.1643.

Example 23

1-Cyclopropyl-6-fluoro-1,4-dihydro-7-(3-methyl-3,6-diazabicyclo[3.2.1]oct-6-yl)-4-oxo-3-quinoline carboxylic Acid

A mixture of 300 mg (1.13 mmol) of 1-cyclopropyl-6,7-difluoro-1,4-dihydro-4-oxo-3-quinolinecarboxylic acid, 652 mg (2.26 mmol) of 3-methyl-3,6-diazabicyclo[3.2.1]octane dihydrobromide, 160 mg (5.65 mmol) of 1,5-diazabicyclo[5.4.0]undec-5-ene (DBU), and 4 ml of pyridine was heated to 80°C for 3 hours. The solvent was removed by rotary evaporation and the residue was dissolved in $CHCl_3$ and extracted twice with aqueous 1N HCl solution. Following a $CHCl_3$ backwash, the pH of the combined acidic extracts was adjusted to 8.5 with aqueous $NaHCO_3$ solution and extracted with $CHCl_3$. The $CHCl_3$ extracts were dried ($Na_2SO_4$) and evaporated to an oil which was crystallized from isopropanol to provide 211 mg (50%) of 1-cyclopropyl-6-fluoro-1,4-dihydro-7-(3-methyl-3,6-diazabicyclo[3.2.1]oct-6-yl)-4-oxo-3- quinolinecarboxylic acid, mp 207.5-208.5°C; $^1H$-NMR (300MHz, $CDCl_3$) δ 1.1-1.4 (6H, m), 1.66 (1H, d, J=12), 2.21 (3H, s), 2.5-2.6 (1H, m), 2.84 (1H, d, J=12), 3.08 (1H, d, J=12), 4.45 (1H, s), 6.85 (1H, d, J=7), 7.82 (1H, d, J=13), 8.57 (1H, s); HRMS, found 371.1640, calcd. for $C_{20}H_{22}N_3O_3F$ 371.1646.

Example 24

The compounds of Examples 5,6,9,10,15,16,22 and 23 were tested for antibacterial activity and were found to be active against Staphylococcus aureus, Staphylococcus epidermus, Streptococcus pyogenes, E. coli, Klebsiella, Pasteurella, Serratia, and Neisseria gonorrhea at levels lower than 15 micrograms per ml.

**Claims**

1. A compound of the formula

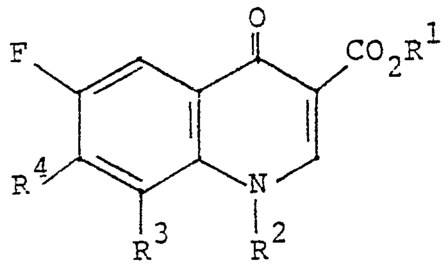

or a pharmaceutically acceptable acid addition salt thereof, wherein
$R^1$ is hydrogen, $(C_1-C_6)$alkyl, or a pharmaceutically acceptable cation;
$R^2$ is ethyl, fluoroethyl, p-fluorophenyl, p-hydroxyphenyl or cyclopropyl;
$R^3$ is fluorine or hydrogen; and
$R^4$ is

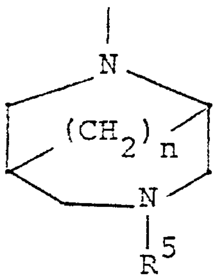

(a)

or

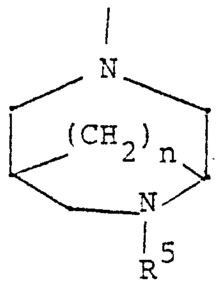

(b)

wherein n is 1 or 2 and $R^5$ is hydrogen or $(C_1-C_3alkyl)$.

2. A compound according to claim 1, wherein $R^1$ and $R^3$ are hydrogen and $R^5$ is methyl.
3. A compound according to claim 1, wherein $R^1$ and $R^3$ are hydrogen and $R^5$ is cyclopropyl.
4. A compound according to claim 1, said compound being selected from the group consisting of

1-cyclopropyl-6-fluoro-1,4-dihydro-7-(6-methyl-3,6-diazabicyclo[3.2.2]non-3-yl)-4-oxo-3-quinoline carboxylic acid;

1-ethyl-6-fluoro-1,4-dihydro-7-(6-methyl-3,6-diazabicyclo[3.2.2]non-3-yl)-4-oxo-3-quinolinecarboxylic acid;

1-cyclopropyl-6-fluoro-1,4-dihydro-7-(3-methyl-3,6-diazabicyclo[3.2.2]non-6-yl)-4-oxo-3-quinolinecarboxylic acid;

1-ethyl-6-fluoro-1,4-dihydro-7-(3-methyl-3-diazabicyclo[3.2.2]non-6-yl)-4-oxo-3-quinolinecarboxylic acid;

1-ethyl-6-fluoro-1,4-dihydro-7-(3-methyl-3,6-diazabicyclo[3-2.1]oct-6-yl)-4-oxo-3-quinolinecarboxylic acid;

1-cyclopropyl-6-fluoro-1,4-dihydro-7-(3-methyl-3,6-diazabicyclo[3.2.1]oct-6-yl)-4-oxo-3-quinoline carboxylic acid;

1-ethyl-6-fluoro-1,4-dihydro-7-(6-methyl-3,6-diazabicyclo[3.2.1]oct-3-yl)-4-oxo-3-quinolinecarboxylic acid;

1-cyclopropyl-6-fluoro-1,4-dihydro-7-(6-methyl-3, 6-diazabicyclo[3.2.1]oct-3-yl)-4-oxo-3-quinolinecarboxylic acid; and

pharmaceutically acceptable acid addition salts of the foregoing compounds.

5. An antibacterial composition comprising a compound according to any one of the preceding claims in an amount sufficient for treatment of a bacterial infection, and a pharmaceutically acceptable carrier.
6. A compound according to any one of claims 1 to 4, for use in medicine.
7. Use of a compound according to any one of claims 1 to 4 for manufacture of a medicament for treating bacterial infections.
8. A compound of the formula

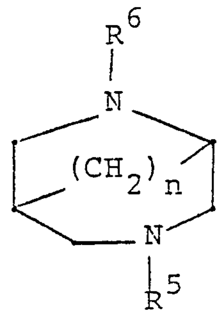 VI

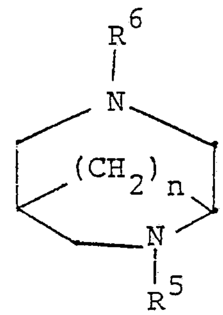 VIII

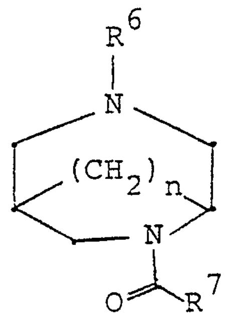 X

or

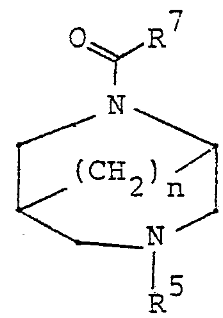 XIII

wherein n is 1 or 2; $R^5$ is hydrogen or $(C_1\text{-}C_3)$alkyl; $R^6$ is hydrogen or a group easily removed under reductive conditions; and $R^7$ is hydrogen, $(C_1\text{-}C_4)$alkyl or $(C_1\text{-}C_4)$alkoxy.

9. A compound according to claim 8, wherein $R^6$ is hydrogen, benzyl, para-nitro benzyl or paramethoxy benzyl.

10. A compound according to claim 9, wherein $R^6$ is hydrogen or benzyl.

**Claims for the following Contracting State: ES**

1. A process for preparing a compound of the formula

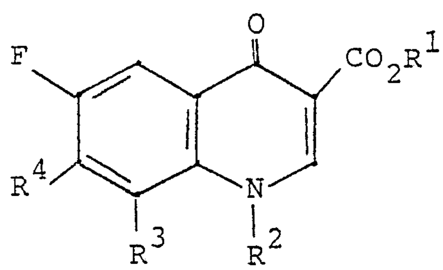

or a pharmaceutically acceptable acid addition salt thereof, wherein

$R^1$ is hydrogen, $(C_1\text{-}C_6)$alkyl, or a pharmaceutically acceptable cation;

$R^2$ is ethyl, fluoroethyl, p-fluorophenyl, p-hydroxyphenyl or cyclopropyl;

$R^3$ is fluorine or hydrogen; and

$R^4$ is

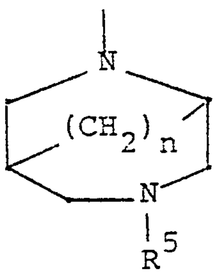

or

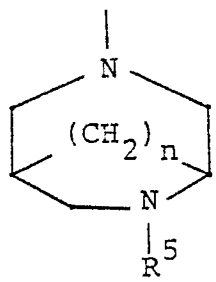

(a) (b)

wherein n is 1 or 2 and $R^5$ is hydrogen or ($C_1$-$C_3$)alkyl, comprising reacting a compound of the formula

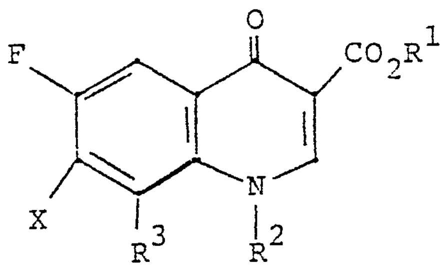

II

wherein $R^1$, $R^2$ and $R^3$ are as defined above and X is halogen with a compound of the formula $R^4H$ wherein $R^4$ is as defined above, and, if desired, preparing the acid addition salt.

2. A process according to claim 1, wherein $R^1$ and $R^3$ are hydrogen and $R^5$ is methyl.

3. A process according to claim 1, wherein $R^1$ and $R^3$ are hydrogen and $R^5$ is cyclopropyl.

4. A process according to claim 1, wherein said compound is selected from the group consisting of

1-cyclopropyl-6-fluoro-1,4-dihydro-7-(6-methyl-3,6-diazabicyclo[3.2.2]non-3-yl)-4-oxo-3-quinolinecarboxylic acid;

1-ethyl-6-fluoro-1,4-dihydro-7-(6-methyl-3,6-diazabicyclo[3.2.2]non-3-yl)-4-oxo-3-quinolinecarboxylic acid;

1-cyclopropyl-6-fluoro-1,4-dihydro-7-(3-methyl-3,6-diazabicyclo[3.2.2]non-6-yl)-4-oxo-3-quinolinecarboxylic acid;

1-ethyl-6-fluoro-1,4-dihydro-7-(3-methyl-3,6-diazabicyclo[3.2.2]non-6-yl)-4-oxo-3-quinolinecarboxylic acid;

1-ethyl-6-fluoro-1,4-dihydro-7-(3-methyl-3,6-diazabicyclo[3.2.1]oct-6-yl)-4-oxo-3-quinolinecarboxylic acid;

1-cyclopropyl-6-fluoro-1,4-dihydro-7-(3-methyl-3,6-diazabicyclo[3.2.1]oct-6-yl)-4-oxo-3-quinoline carboxylic acid;

1-ethyl-6-fluoro-1,4-dihydro-7-(6-methyl-3,6-diazabicyclo[3.2.1]oct-3-yl)-4-oxo-3-quinolinecarboxylic acid;

1-cyclopropyl-6-fluoro-1,4-dihydro-7-(6-methyl-3, 6-diazabicyclo[3.2.1]oct-3-yl)-4-oxo-3-quinolinecarboxylic acid; and

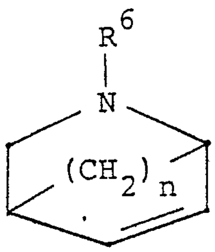

V

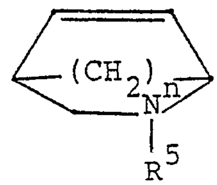

VII

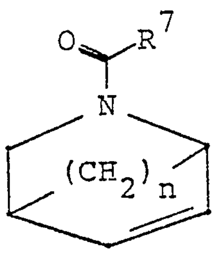

IX

or

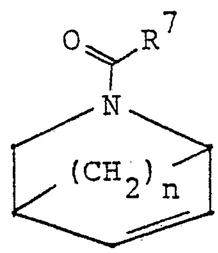

IX

respectively, wherein $R^6$, $R^5$ and $R^7$ are as defined above, with a compound of the formula $R^5NH_2$ , $R^6NH_2$ , $R^6NH_2$ or $R^5NH_2$, respectively, wherein $R^5$ and $R^6$ as defined above, and, if desired,

(a) reducing a compound of the formula VI or VIII, wherein $R^6$ is other than hydrogen and $R^5$ is ($C_1$-$C_3$)alkyl, to a compound of the formula VI or VIII, respectively, wherein $R^6$ is hydrogen;

(b) reducing a compound of the formula X

(i) wherein $R^7$ is ($C_1$-$C_2$)alkyl and $R^6$ is as defined above for formula X to prepare a compound of the formula VIII wherein $R^5$ is ($C_2$-$C_3$)alkyl and, if desired, reducing the latter compound to a compound wherein $R^6$ is hydrogen;

(ii) wherein $R^7$ is ($C_1$-$C_4$)alkoxy and $R^6$ is as defined above for formula X to prepare a compound of the VIII wherein $R^5$ is methyl, and, if desired, reducing the latter compound to a compound wherein $R^6$ is hydrogen; or

pharmaceutically acceptable acid addition salts of the foregoing compounds.

5. A process for preparing a compound of the formula

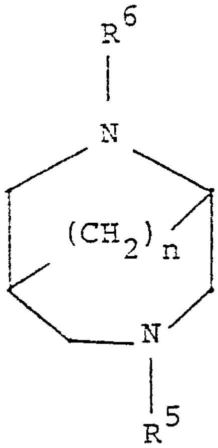
VI

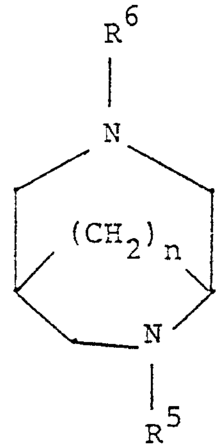
VIII

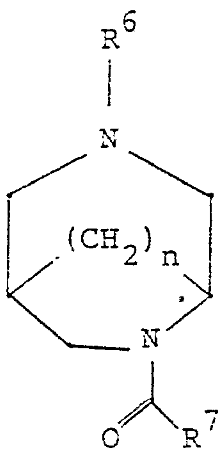
X

or

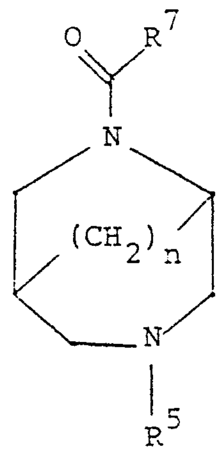
XIII

wherein n is 1 or 2; $R^5$ is hydrogen or ($C_1$-$C_3$) alkyl; $R^6$ is hydrogen or a group easily removed under reductive conditions; and $R^7$ is hydrogen, ($C_1$-$C_4$) alkyl or ($C_1$-$C_4$) alkoxy, comprising reacting a compound of the formula

(c) hydrolyzing a compound of the formula XIII wherein $R^5$ and $R^7$ are as defined above for formula XIII to prepare a compound of the formula VI wherein $R^6$ is hydrogen.

6. A process according to claim 5, wherein said compound of the formula VI is prepared by reacting said compound of the formula V with said compound of the formula $R^5NH_2$.

7. A process according to claim 5, wherein said compound of the formula VIII is prepared by reacting said compound of the formula VII with said compound of the formula $R^6NH_2$.

8. A process according to claim 5, wherein said compound of the formula X is prepared by reacting said compound of the formula IX with said compound of the formula $R^6NH_2$

9. A process according to claim 5, wherein said compound of the formula XIII is prepared by reacting said compound of the formula IX with said compound of the formula $R^5NH_2$.

10. A process according to claim 5, wherein $R^6$ hydrogen, benzyl, para-nitro benzyl or paramethoxy benzyl.

**Claims for the following Contracting State: GR**

1. A process for preparing a compound of the formula

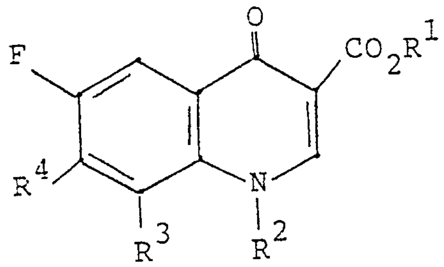

or a pharmaceutically acceptable acid addition salt thereof, wherein
$R^1$ is hydrogen, ($C_1$-$C_6$) alkyl, or a pharmaceutically acceptable cation;
$R^2$ is ethyl, fluoroethyl, p-fluorophenyl, p-hydroxyphenyl or cyclopropyl;
$R^3$ is fluorine or hydrogen; and
$R^4$ is

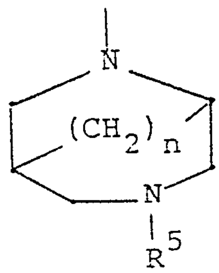 (a) or 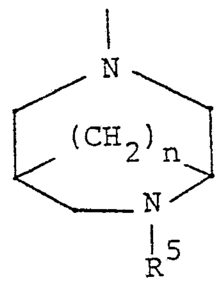 (b)

wherein n is 1 or 2 and $R^5$ is hydrogen or ($C_1$-$C_3$) alkyl, comprising reacting a compound of the formula

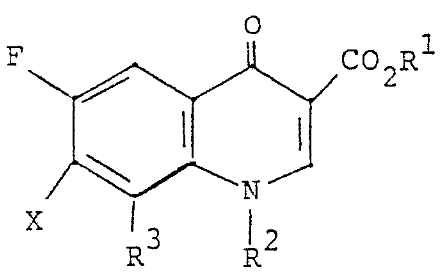 II

wherein $R^1$, $R^2$ and $R^3$ are as defined above and X is halogen with a compound of the formula $R^4H$ wherein $R^4$ is as defined above, and, if desired, preparing the acid addition salt.

2. A process according to claim 1, wherein $R^1$ and $R^3$ are hydrogen and $R^5$ is methyl.

3. A process according to claim 1, wherein $R^1$ and $R^3$ are hydrogen and $R^5$ is cyclopropyl.

4. A process according to claim 1, wherein said compound is selected from the group consisting of

1-cyclopropyl-6-fluoro-1,4-dihydro-7-(6-methyl-3,6-diazabicyclo[3.2.2]non-3-yl)-4-oxo-3-quinolinecarboxylic acid;

1-ethyl-6-fluoro-1,4-dihydro-7-(6-methyl-3,6-diazabicyclo[3.2.2]non-3-yl)-4-oxo-3-quinolinecarboxylic acid;

1-cyclopropyl-6-fluoro-1,4-dihydro-7-(3-methyl-3,6-diazabicyclo[3.2.2]non-6-yl)-4-oxo-3-quinolinecarboxylic acid;

1-ethyl-6-fluoro-1,4-dihydro-7-(3-methyl-3,6-diazabicyclo[3.2.2]non-6-yl)-4-oxo-3-quinolinecarboxylic acid;

1-ethyl-6-fluoro-1,4-dihydro-7-(3-methyl-3,6-diazabicyclo[3.2.1]oct-6-yl)-4-oxo-3-quinolinecarboxylic acid;

1-cyclopropyl-6-fluoro-1,4-dihydro-7-(3-methyl-3,6-diazabicyclo[3.2.1]oct-6-yl)-4-oxo-3-quinolinecarboxylic acid;

1-ethyl-6-fluoro-1,4-dihydro-7-(6-methyl-3,6-diazabicyclo[3.2.1]oct-3-yl)-4-oxo-3-quinolinecarboxylic acid;

1-cyclopropyl-6-fluoro-1,4-dihydro-7-(6-methyl-3, 6-diazabicyclo[3.2.1]oct-3-yl)-4-oxo-3-quinoline-

carboxylic acid; and

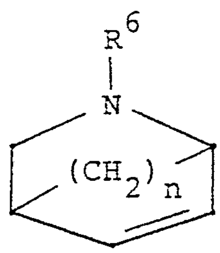

V

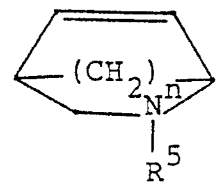

VII

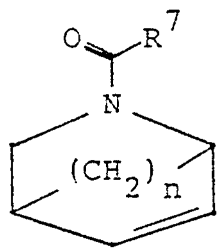

IX

or

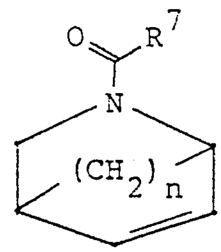

IX

respectively, wherein $R^6$, $R^5$ and $R^7$ are as defined above, with a compound of the formula $R^5NH_2$, $R^6NH_2$, $R^6NH_2$ or $R^5NH_2$, respectively, wherein $R^5$ and $R^6$ are as defined above, and, if desired,

(a) reducing a compound of the formula VI or VIII, wherein $R^6$ is other than hydrogen and $R^5$ is $(C_1$-$C_3)$ alkyl, to a compound of the formula VI or VIII, respectively, wherein $R^6$ is hydrogen;

(b) reducing a compound of the formula X

(i) wherein $R^7$ is $(C_1$-$C_2)$ alkyl and $R^6$ is as defined above for formula X to prepare a compound of the formula VIII wherein $R^5$ is $(C_2$-$C_3)$ alkyl and, if desired, reducing the latter compound to a compound wherein $R^6$ is hydrogen;

(ii) wherein $R^7$ is $(C_1$-$C_4)$ alkoxy and $R^6$ is as defined above for formula X to prepare a compound of the VIII wherein $R^5$ is methyl, and, if desired, reducing the latter compound to a compound wherein $R^6$ is hydrogen; or

pharmaceutically acceptable acid addition salts of the foregoing compounds.

5. A process for preparing a compound of the formula

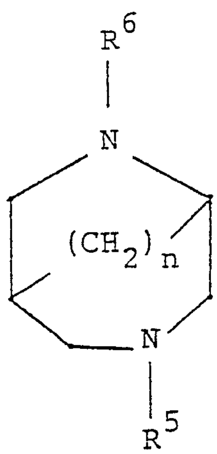

VI

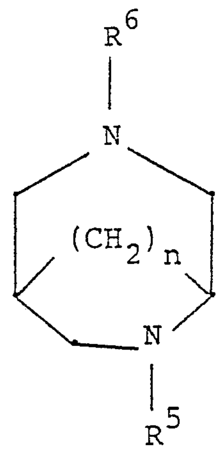

VIII

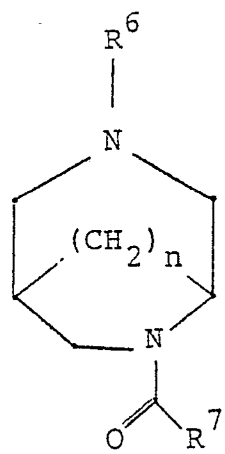

X

or

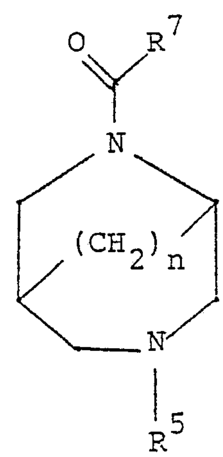

XIII

wherein n is 1 or 2; $R^5$ is hydrogen or $(C_1\text{-}C_3)$ alkyl; $R^6$ is hydrogen or a group easily removed under reductive conditions; and $R^7$ is hydrogen, $(C_1\text{-}C_4)$ alkyl or $(C_1\text{-}C_4)$ alkoxy, comprising reacting a compound of the formula

(c) hydrolyzing a compound of the formula XIII wherein $R^5$ and $R^7$ are as defined above for formula XIII to prepare a compound of the formula VI wherein $R^6$ is hydrogen.

6. A process according to claim 5, wherein said compound of the formula VI is prepared by reacting said compound of the formula V with said compound of the formula $R^5NH_2$.

7. A process according to claim 5, wherein said compound of the formula VIII is prepared by reacting said compound of the formula VII with said compound of the formula $R^6NH_2$.

8. A process according to claim 5, wherein said compound of the formula X is prepared by reacting said compound of the formula IX with said compound of the formula $R^6NH_2$.

9. A process according to claim 5, wherein said compound of the formula XIII is prepared by reacting said compound of the formula IX with said compound of the formula $R^5NH_2$.

10. A process according to claim 5, wherein $R^6$ is hydrogen, benzyl, para-nitro benzyl or paramethoxy benzyl.

11. A compound of the formula

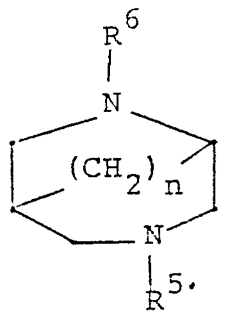 VI

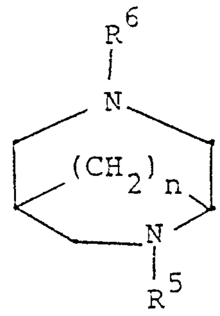 VIII

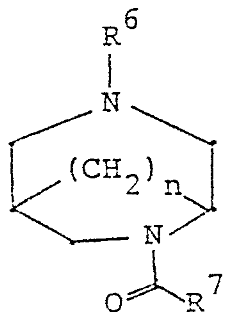 X

or

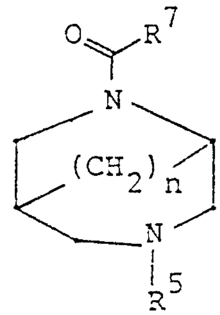 XIII

wherein n is 1 or 2; $R^5$ is hydrogen or $(C_1\text{-}C_3)$alkyl; $R^6$ is hydrogen or a group easily removed under reductive conditions; and $R^7$ is hydrogen, $(C_1\text{-}C_4)$alkyl or $(C_1\text{-}C_4)$alkoxy.

12. A compound according to claim 11,wherein $R^6$ is hydrogen, benzyl, para-nitro benzyl or paramethoxy benzyl.

13. A compound according to claim 12,wherein $R^6$ is hydrogen or benzyl.